(19) Europäisches Patentamt
European Patent Office
Office européen des brevets

(11) Veröffentlichungsnummer: **0 013 741**
**B1**

(12) **EUROPÄISCHE PATENTSCHRIFT**

(45) Veröffentlichungstag der Patentschrift: **16.09.81**

(51) Int. Cl.³: **C 07 D 307/60** // B01J27/18, B01J37/00

(21) Anmeldenummer: **79105180.8**

(22) Anmeldetag: **14.12.79**

(54) **Verfahren zur Herstellung von Maleinsäureanhydrid.**

(30) Priorität: **26.01.79 DE 2902986**

(43) Veröffentlichungstag der Anmeldung: **06.08.80 Patentblatt 80/16**

(45) Bekanntmachung des Hinweises auf die Patenterteilung: **16.09.81 Patentblatt 81/37**

(84) Benannte Vertragsstaaten: **BE FR GB IT**

(56) Entgegenhaltungen:
**DE-A-2 138 692**
**DE-A-2 351 151**
**FR-A-2 072 336**
**GB-A-1 157 117**

(73) Patentinhaber: **CHEMISCHE WERKE HÜLS AG, Postfach 1320, D-4370 Marl 1 (DE)**

(72) Erfinder: **Franz, Gerhard, Dr., Gersthofener Strasse 18, D-4370 Marl (DE)**
Erfinder: **Ratajczak, Hans-Josef, Dr., Gersthofener Strasse 14, D-4370 Marl (DE)**
Erfinder: **Nierlich, Franz, Dr., Bitterfelder Strasse 9 a, D-4370 Marl (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 013 741 B1

ACTORUM AG.

# Verfahren zur Herstellung von Maleinsäureanhydrid

Maleinsäureanhydrid kann man durch Oxidation eines gesättigten oder ungesättigten $C_4$-Kohlenwasserstoffs in Gegenwart geeigneter Katalysatoren herstellen. Die bekannten Verfahren unter Einsatz von Vanadin-Phosphor-Katalysatoren haben jedoch den Nachteil, dass die erzielten Ausbeuten an Maleinsäureanhydrid zu niedrig sind (US-PS 3 474 041; US-PS 3 618 959). Daneben besitzen Vanadin-Phosphorsäure-Kontakte nur eine geringe Kontaktstandzeit, da durch Austrag von Phosphorsäure und Reoxidation von 4-wertigem Vanadium die Selektivität deutlich zurückgeht. Durch kontinuierliches oder diskontinuierliches Eindosieren flüchtiger Phosphorverbindungen in den Prozessgasstrom kann die Standzeit nur bedingt verlängert werden.

In der US-PS 4 021 417 werden Molybdän-Antimon-Katalysatoren beschrieben, die zur Butadienoxidation eingesetzt werden. Die Herstellung dieser Katalysatoren erfolgt durch Erhitzen einer Aufschlämmung der Ausgangsverbindungen. Durch Zugabe einer metallischen Komponente soll eine partielle Reduktion des 6-wertigen Molybdäns erreicht werden.

Verfahren, die unter Verwendung dieser Katalysatoren durchgeführt werden, sind jedoch hinsichtlich der Reproduzierbarkeit der Kontaktherstellung und der Selektivität bezüglich Maleinsäureanhydrid unbefriedigend.

Aufgabe der vorliegenden Erfindung ist daher die Schaffung eines Verfahrens zur Oxidation von ungesättigten n-$C_4$-Kohlenwasserstoffen zu Maleinsäureanhydrid in Gegenwart von Katalysatoren, die reproduzierbar hergestellt werden können, eine lange Lebensdauer besitzen und mit sehr hoher Selektivität zu Maleinsäureanhydrid führen.

Die Aufgabe wurde dadurch gelöst, dass man ein Gemisch aus ungesättigten n–$C_4$-Kohlenwasserstoffen und einem sauerstoffhaltigen Gas bei Temperaturen von 300 bis 450 °C und bei einer scheinbaren Kontaktzeit von 0,1 bis 30 Sekunden mit einem Katalysator in Berührung bringt, der die Formel

$$W_aMo_bSb_cY_dX_eO_x$$

hat, wobei Y Phosphor und/oder Silicium ist und X ein Element der Gruppe Vanadin, Wismuth, Tellur und/oder ein Alkalimetall sein kann und a und b Zahlen zwischen 1 und 12 sind, c eine Zahl zwischen 2 und 20, d eine Zahl zwischen 0,2 und 2, e eine Zahl zwischen 0 und 10 und x die Zahl der Sauerstoffatome ist, die für die Absättigung der restlichen Valenzen der übrigen vorhandenen Elemente erforderlich ist und den man durch Zusammengeben einer Verbindung des dreiwertigen Antimons sowie gegebenenfalls einer Vanadin-, Wismuth-, Tellur- und/oder Alkaliverbindung mit einer Lösung der Heteropolysäuren des Molybdäns und Wolframs bei einer Temperatur von 0 bis 200 °C, anschliessende Trocknung und Temperung bei einer Temperatur von 300 bis 500 °C erhält. Zur Herstellung des Kontaktes verwendet man als Ausgangssubstanzen Heteropolysäuren des Molybdäns und Wolframs. Bei Zugabe von dreiwertigem Antimon zur homogenen Lösung dieser Heteropolysäuren erfolgt die Bildung eines tiefblauen Katalysatorkomplexes, der nach Abziehen des Lösungsmittels, Calcinieren und Verformen direkt in die Oxidation eingesetzt werden kann.

Bei der Herstellung dieser Kontakte ist die Verwendung von Heteropolysäuren des Molybdäns und Wolframs in Kombination mit Verbindungen des dreiwertigen Antimons wesentlich.

Die Heteropolysäuren stellen eindeutig definierte, gut reproduzierbare Verbindungen dar, die sich durch sehr gute Löslichkeit in Wasser und vielen sauerstoffhaltigen organischen Lösungsmitteln auszeichnen. Zudem liegen die Schwermetalle in den Heteropolysäuren in redoxlabiler Form vor.

Bei Zugabe einer Verbindung des dreiwertigen Antimons zur Lösung einer Molybdän-Heteropolysäure erfolgt die Bildung eines tiefblauen Gels. Dieses Verhalten kann auf die partielle Reduktion des 6-wertigen Molybdäns durch das 3-wertige Antimon zurückgeführt werden. Das Trocknen und Calcinieren eines solchen Gels liefert bereits gute Katalysatoren. Deutlich bessere Katalysatoren werden jedoch erhalten, wenn anstelle der Lösung der reinen Mo-Heteropolysäure eine Lösung der Heteropolysäuren von Molybdän und Wolfram eingesetzt wird und zu dieser eine Verbindung des dreiwertigen Antimons gegeben wird. Da man mit den Wolfram-Heteropolysäuren allein mit Antimon völlig unbefriedigende Ergebnisse erhält, ist dies auf einen synergistischen Effekt zurückzuführen. Die zusätzliche Anwesenheit von Vanadin, Wismuth, Tellur und/oder Alkali kann günstig sein.

Als Heteropolysäuren des Molybdäns und Wolframs setzt man bevorzugt solche mit Phosphor oder Silicium als Heteroatom ein. Beispiele solcher Heteropolysäuren sind:

$H_3[PMo_{12}O_{40}]$, $H_{12}[P_2Mo_{18}O_{65}]$, $H_3[PW_{12}O_{40}]$, $H_3[PW_2Mo_{10}O_{40}]$, $H_3[PW_6Mo_6O_{40}]$, $H_8[SiW_{12}O_{42}]$, $H_8[SiMo_{12}O_{42}]$.

Die Herstellung der Heteropolysäuren erfolgt z.B. nach den in der Literatur bekannten Methoden: 1. Drechselsche Äthermethode, 2. doppelte Umsetzung von Salzen, 3. Erhitzen der Ausgangsoxide (gegebenenfalls unter Druck) in Wasser.

Das dreiwertige Antimon kann in beliebiger Form eingesetzt werden, bevorzugt als Halogenid oder Oxid.

Die Elemente Vanadin, Wismuth, Tellur und/oder die Alkalimetalle werden bevorzugt als Nitrat oder Oxid eingesetzt.

Bei der Herstellung der Katalysatoren stellt man im allgemeinen zunächst eine homogene Lösung der Heteropolysäuren her. Als Lösungsmittel eignen sich Wasser bzw. sauerstoffhaltige organische Substanzen, wie zum Beispiel Ethanol oder iso-Propanol. Zu dieser Lösung gibt man unter gutem Rühren portionsweise oder auf einmal eine Verbindung des dreiwertigen Antimons. Ohne Einfluß auf die Qualität des Katalysators kann allerdings auch zur Lösung oder Suspension einer dreiwertigen Antimonverbindung die Heteropolysäure oder ein Gemisch derselben hinzugegeben werden.

Das bzw. die Elemente der Gruppe X können zu einem beliebigen Zeitpunkt dem Reaktionsgemisch hinzugefügt werden.

Die Temperatur der Reaktionslösung liegt zwischen 0 und 200 °C. Bei hohen Temperaturen ist jedoch die Anwendung von Druck erforderlich. Bevorzugte Temperaturen liegen zwischen 30 und 110 °C.

Die Reaktionszeiten bei der Katalysatorherstellung können zwischen wenigen Minuten und einigen Stunden variieren. Bevorzugte Zeiten liegen zwischen 15 Minuten und 2 Stunden.

Wesentlich für die Durchführung der Reaktion ist die Bildung des blauen, gelartigen Katalysator-Vorläufers. Die genaue Struktur ist nicht bekannt, man kann jedoch davon ausgehen, dass das dreiwertige Antimon in partiell oxidierter und das 6-wertige Molybdän bzw. Wolfram in partiell reduzierter Form vorliegen. Die Bildung eines solchen Vorläufers ist entscheidend für die sehr gute Reproduzierbarkeit der Katalysatoren.

Nach Bildung des Vorläufers, erkenntlich an der intensiv blauen Farbe, wird das Lösungsmittel abgedampft bzw. abgezogen. Der Rückstand wird bei erhöhter Temperatur getrocknet. Geeignete Temperaturen liegen zwischen 100 und 200 °C. Das getrocknete Material wird gemörsert bzw. gemahlen und zu Formkörpern verarbeitet. Eine Möglichkeit der Verarbeitung ist das Verpressen zu Pillen. Häufig erfordern derartige Konfektionierungsmethoden die Zugabe von Gleitmitteln. Als solche können Graphit, Stearinsäure und ähnliche eingesetzt werden.

Der geformte Katalysator wird anschliessend bei einer Temperatur von 300 bis 500 °C calciniert. Vorzugsweise aktiviert man den Katalysator bei einer Temperatur von etwa 400 bis 450 °C für etwa 1 bis 4 Stunden im Luftstrom. Entsprechend einer gleichwertigen Methode wird das Katalysatormaterial vor dem Verarbeiten zu Formkörpern calciniert.

Der erfindungsgemässe Katalysator kann auch auf einem Trägermaterial aufgebracht sein. Als solches eignet sich Titandioxid, Aluminiumoxid, Ton, Diatomäenerde und dergleichen, die man in Mengen von 2 bis 50 Gewichtsprozent, bezogen auf das Gesamtgewicht des Katalysators, anwendet. Der Träger kann der Katalysatorlösung zugegeben werden, oder die Katalysatorlösung kann auf den Träger gegossen werden. Desgleichen kann der Träger während des ganzen Verlaufs der Präparation anwesend sein.

Als ungesättigte n-$C_4$-Kohlenwasserstoffe eignen sich Buten-1, Buten-2 und insbesondere Butadien. Mit Butadien erhält man die höchsten Ausbeuten an Maleinsäureanhydrid.

Zur Herstellung von Maleinsäureanhydrid wird ein Gemisch aus einem ungesättigten n–$C_4$-Kohlenwasserstoff und einem sauerstoffhaltigen Gas über den erfindungsgemässen Katalysator geleitet. Luft ist als Sauerstoffquelle zufriedenstellend, jedoch können synthetische Mischungen aus Sauerstoff und Verdünnungsgasen, wie Stickstoff, ebenfalls verwendet werden. Auch mit Sauerstoff angereicherte Luft kann eingesetzt werden.

Das Verhältnis von Sauerstoff zu dem ungesättigten n-$C_4$-Kohlenwasserstoff kann im Bereich von etwa 2:1 bis etwa 4:1 und vorzugsweise im Bereich von 4:1 bis 20:1 liegen.

Die Ofentemperatur liegt zwischen 300 und 450 °C, bevorzugt zwischen 330 und 420 °C. Temperaturen oberhalb 420 °C sind weniger günstig, da dann bereits Weiteroxidation von gebildetem Maleinsäureanhydrid beobachtet wird.

Die Reaktion erfolgt im allgemeinen bei leichtem Überdruck. Man kann jedoch auch bei Normaldruck oder leichtem Unterdruck arbeiten.

Die Reaktion kann im Festbett oder im Wirbelbett durchgeführt werden. Beim Arbeiten im Festbett sind Rohrbündelreaktoren einzusetzen, um die freiwerdende Reaktionswärme ausreichend abführen zu können.

Die scheinbare Kontaktzeit liegt im Bereich von 0,1 bis 30 Sekunden und beträgt vorzugsweise etwa 0,2 bis 10 Sekunden. Die scheinbare Kontaktzeit bezieht sich auf das Leerrohr.

Gegebenenfalls kann man auch Wasserdampf zusetzen. Dampfmengen im Bereich von 0 bis 20 Vol-% bezogen auf das Einsatzgemisch führen zu keiner Schädigung des Katalysators.

Die nach dem erfindungsgemässen Verfahren hergestellten Katalysatoren zeichnen sich durch eine sehr lange Lebensdauer aus. Bei Standzeitversuchen im Technikumsmassstab wurde nach einer Betriebszeit von 2 Jahren kein Verlust an Aktivität und Selektivität beobachtet.

Überraschenderweise wurde bei den erfindungsgemässen Katalysatoren kein Austrag von Phosphorverbindungen bzw. Molybdänverbindungen beobachtet. Dies ist insbesondere deshalb überraschend, weil bei den V-P-Kontakten das Problem des Phosphoraustrages zu den gravierenden Nachteilen gehört.

Zudem haben die anspruchsgemäss verwendeten Katalysatoren eine aussergewöhnlich gute mechanische Beständigkeit, da nach einer Betriebszeit von 2 Jahren kaum Staubanfall beobachtet wurde. Dieses Verhalten lässt den Schluss auf wesentlich höhere Standzeiten zu.

Das bei der Reaktion gebildete Maleinsäureanhydrid wird in sehr reiner Form erhalten. Beim Durchleiten des Produktstromes durch ein gekühltes Fallensystem schlägt es sich in Form weisser Kristalle nieder. Die wesentlichen Nebenprodukte sind Kohlendioxid und Kohlenmonoxid. In kleineren Mengen fallen Essigsäure und Acrylsäure an. Die in den Tabellen I und II aufgeführten Katalysatoren wurden gemäss den folgenden Beispielen hergestellt.

Vergleichsbeispiel 1

$P_{1,4}SB_{11,4}W_{4,8}Mo_{12}O_x$

19,64 g 5 $(NH_4)_2O \cdot 12WO_3 \cdot 7H_2O$ werden in 1,2 l $H_2O$ unter Erwärmen gelöst und danach auf 400 ml eingedampft. Man gibt 33,04 g $(NH_4)_6Mo_7O_{40} \cdot 4H_2O$ und 2,52 g $H_3PO_4$ hinzu und erhitzt bis wiederum eine klare Lösung entsteht. In obige Lösung gibt man 25,70 g $Sb_2O_3$ und rührt weitere 2 Stunden bei 100 °C. Danach dampft man das Wasser ab und trocknet den Rückstand über Nacht bei 120 °C im Trockenschrank. Die feste Masse wird pulverisiert und mit 2% Graphit zu Pillen verpresst. Der Katalysator wird vor dem Einsatz 2 Stunden bei 430 °C im Luftstrom aktiviert.

In einem 25 ml-Fixbett-Glasreaktor mit einem Innendurchmesser von 2,0 cm füllt man 25 ml des Katalysators ein und beheizt den Reaktor mit einem Salzbad der in den Tabellen angegebenen Badtemperatur. Bei dieser Badtemperatur leitet man mit einem leichten Vordruck von 0,1 bar Butadien-1,3 und Luft durch das Katalysatorbett bei den in den Tabellen angegebenen scheinbaren Kontaktzeiten und Konzentrationen. Die Dosierung der gasförmigen Reaktionskomponenten erfolgt über elektronische Mengenregler.

Vergleichsbeispiel 2

$P_1Sb_8Mo_{12}O_x$

40,0 $H_3[PMo_{12}O_{40}]$ werden in 100 ml Wasser gelöst. Bei 50 °C gibt man 23,32 g $Sb_2O_3$ unter heftigem Rühren auf einmal hinzu. Es entsteht ein tiefblaues Gel, das noch weitere 2 Stunden bei 80 °C gerührt wird. Danach dampft man das Wasser ab bis eine feste Masse entsteht und trocknet diese 10 Stunden bei 135 °C im Trockenschrank. Der Kuchen wird gemörsert, mit 2% Graphit versetzt und zu Pillen verpresst. Vor dem Einsatz wird der Katalysator 2 Stunden bei 430 °C im Luftstrom calciniert.

Die Oxidation von Butadien erfolgt nach den Angaben des Vergleichsbeispiels 1.

Beispiel 1

$P_{1,4}Sb_{11,4}W_{4,8}Mo_{12}O_x$

Zu einer Lösung von 31,26 g $H_3[PMo_{12}O_{40}]$ und 18,76 g $H_3(PW_{12}O_{40}]$ in 150 ml Wasser gibt man bei 50 °C unter kräftigem Rühren 25,66 g $Sb_2O_3$ auf einmal zu. Das entstehende dunkelblaue Gel wird weitere 2 Stunden bei 90 °C gerührt. Danach dampft man das Wasser ab und trocknet den Rückstand 5 Stunden bei 135 °C. Der feste Kuchen wird gemörsert, mit 2% Graphit versetzt und zu Tabletten verpresst. Diese werden 2 Stunden bis 430 °C im Luftstrom calciniert.

In einem 25 ml-Fixbett-Glasreaktor mit einem Innendurchmesser von 2,0 cm füllt man 25 ml des Katalysators ein und beheizt den Reaktor mit einem Salzbad der in den Tabellen angegebenen Badtemperatur. Bei dieser Badtemperatur leitet man mit einem leichten Vordruck von 0,1 bar Butadien-1,3 und Luft durch das Katalysatorbett bei den in den Tabellen angegebenen scheinbaren Kontaktzeiten und Konzentrationen. Die Dosierung der gasförmigen Reaktionskomponenten erfolgt über elektronische Mengenregler.

Beispiel 2

Der Katalysator ist der gleiche, wie in Beispiel 1. Die Oxidation von Butadien erfolgt nach den Angaben des Beispiels 1.

Beispiel 3

$P_{1,4}Sb_{11,4}Bi_{0,26}W_{4,8}Mo_{12}O_x$

Zu einer Lösung von 18,76 g $H_3[PMo_{12}O_{40}]$ und 11,26 g $H_3[PW_{12}O_{40}]$ in 70 ml Wasser gibt man bei 50 °C unter kräftigem Rühren 15,04 g $Sb_2O_3$ und 1,22 g $Bi(NO_3)_3 \cdot 5H_2O$ auf einmal zu. Weitere Verfahrensweise siehe Beispiel 1.

Beispiel 4

$P_{1,4}Sb_{11,4}V_{1,1}Cs_{0,6}W_{4,8}Mo_{12}O_x$

Zu einer Lösung von 18,76 g $H_3[PMo_{12}O_{40}]$ und 11,26 g $H_3[PW_{12}O_{40}]$ in 90 ml Wasser gibt man bei 70 °C unter kräftigem Rühren 15,40 g $Sb_2O_3$, 0,91 g $V_2O_5$ und 0,98 g $Cs_2CO_3$ in mehreren Portionen hinzu. Das Gemisch wird zwei Stunden bei 90 °C gerührt. Man erhält eine dunkelblaue, viskose Masse. Diese wird 12 Stunden bei 135 °C im Trokkenschrank getrocknet und danach gemörsert. Das Pulver versetzt man mit 2% Graphit und verpresst es zu Tabletten. Diese werden 2 Stunden bei 430 °C im Luftstrom calciniert. Die Oxidation von Butadien erfolgt nach den Angaben des Beispiels 1.

Beispiel 5

$P_{1,4}Sb_{6,5}V_{0,65}Li_{0,65}W_5Mo_{12}O_x$

Zu einer Lösung von 26,04 g $H_3[PMo_{12}O_{40}]$ und 15,96 g $H_3[PW_{12}O_{40}]$ in 200 ml Wasser gibt man bei 60 °C 11,67 g $Sb_2O_3$, 0,73 g $V_2O_5$ und 0,30 g $Li_2CO_3$ in mehreren Portionen hinzu. Weitere Verfahrensweise siehe Beispiel 1.

Beispiel 6

$P_{1,4}Sb_{11,4}Te_{0,12}W_{4,8}Mo_{12}O_x$

Zu einer Lösung von 31,26 g $H_3[PMo_{12}O_{40}]$ und 18,76 g $H_3[PW_{12}O_{40}]$ in 250 ml $H_2O$ gibt man bei 90 °C 25,66 g $Sb_2O_3$ und 0,16 g $TeO_2$ auf einmal hinzu. Weitere Verfahrensweise siehe Beispiel 1.

Die gesamte Analytik wird auf gaschromatographischem Wege durchgeführt. Zur Bestimmung der MSA-Konzentration im Produktstrom wird dieser über beheizte Leitungen direkt durch die Probenschleife eines geeigneten Gaschromatographen geleitet. Zur Auswertung wird der Stickstoff des Einsatzgemisches als Innerer Standard benutzt. Daneben kann der Produktstrom auch in einem Fallensystem aufgefangen und durch Zuwiegen eines geeigneten Standards analysiert werden.

Die Langzeitversuche werden in einem 200 ml Fixbett-Stahlreaktor durchgeführt, der ebenfalls einen Innendurchmesser von 2,0 cm aufweist.

Die Ergebnisse der Versuche sind in den folgenden Tabellen angegeben. Zur Bestimmung von Umsatz, Ausbeute und Selektivität werden die folgenden Definitionen angewendet:

$$\text{Umsatz} = \frac{\text{Mol an umgesetztem Kohlenwasserstoff}}{\text{Mol an Kohlenwasserstoff in der Beschickung}} \times 100$$

$$\text{Ausbeute \textit{in einem einzigen} Durchgang} = \frac{\text{Mol an gewonnenem Maleinsäureanhydrid}}{\text{Mol an zugeführtem Kohlenwasserstoff}} \times 100$$

$$\text{Selektivität} = \frac{\text{Ausbeute in einem Durchgang}}{\text{Umsatz}} \times 100$$

Durch das Vergleichsbeispiel 1 wird deutlich, dass der Einsatz von löslichen Molybdän- und Wolfram-Verbindungen alleine keine geeignete Methode zur Herstellung guter Katalysatoren ist. Entscheidend ist der Einsatz von Heteropolysäuren. Nur sie sind in der Lage, mit dem 3-wertigen Antimon in Form einer Redox-Reaktion zu reagieren und den blauen Komplex zu bilden.

Das Vergleichsbeispiel 2 zeigt, daß bei Abwesenheit von Wolfram ein wenig reaktiver Katalysator gebildet wird, der auch nicht die gewünschte Selektivität zeigt.

Die Beispiele 1 bis 6 zeigen die hervorragenden Ergebnisse, die bei erfindungsgemässen Vorgehen erhalten werden.

Durch Vergleich der Beispiele 1 und 2 wird die sehr gute Reproduzierbarkeit bei der Katalysatorherstellung verdeutlicht.

In der Tabelle II sind die Ergebnisse eines Langzeitversuchs aufgeführt.

Die Werte zeigen, dass auch nach 2 Jahren kein Verlust an Aktivität und Selektivität auftrat.

Ein Austrag der Katalysatorelemente wird nicht beobachtet.

Tabelle I:
Oxidation von Butadien-1,3 zu Maleinsäureanhydrid

| Vergleichsbeispiel | Katalysator akt. Bestandteile | Vol-% $C_4$ | Bad-Temp. [°C] | scheinbare Kontaktzeit [sec.] | Umsatz [%] | MSA-Ausbeute Mol-% bei einmal. Durchgang | MSA Selektivität [Mol-%] |
|---|---|---|---|---|---|---|---|
| 1 | $P_{1,4}Sb_{11,4}W_{4,8}Mo_{12}O_x$ | 1,3 | 408 | 1,4 | 77,5 | 35,6 | 45,9 |
| 2 | $P_1Sb_8Mo_{12}O_x$ | 1,23 | 410 | 1,4 | 98,5 | 57,1 | 58,0 |
| Beispiel | | | | | | | |
| 1 | $P_{1,4}Sb_{11,4}W_{4,8}Mo_{12}O_x$ | 1,2 | 370 | 1,3 | 100 | 74,8 | 74,8 |
| 2 | $P_{1,4}Sb_{11,4}W_{4,8}Mo_{12}O_x$ | 1,2 | 370 | 1,3 | 100 | 75,3 | 75,3 |
| 3 | $P_{1,4}Sb_{11,4}Bi_{0,26}W_{4,8}Mo_{12}O_x$ | 2,6 | 380 | 2,0 | 100 | 69,0 | 69,0 |
| 4 | $P_{1,4}Sb_{11,4}V_{1,1}Cs_{0,6}W_{4,8}Mo_{12}O_x$ | 1,1 | 385 | 1,2 | 100 | 75,5 | 75,5 |
| 5 | $P_{1,4}Sb_{6,5}V_{0,65}Li_{0,65}W_5Mo_{12}O_x$ | 2,1 | 385 | 2,1 | 100 | 71,6 | 71,6 |
| 6 | $P_{1,4}Sb_{11,4}Te_{0,12}W_{4,8}Mo_{12}O_x$ | 1,2 | 380 | 1,1 | 100 | 70,4 | 70,4 |

Tabelle II:
Langzeitversuch zur Oxidation von Butadien1,3 zu Maleinsäureanhydrid

| Katalysator Beispiel 1 | Laufzeit (Wochen) | Vol-% $C_4$ | Bad-Temp. [°C] | scheinbare Kontaktzeit (sec.) | Umsatz [%] | MSA-Ausbeute Mol-% bei einmaligem Durchgang | Selektivität [Mol-%] |
|---|---|---|---|---|---|---|---|
| $P_{1,4}Sb_{11,4}W_{4,8}Mo_{12}O_x$ | 1 | 1,54 | 376 | 1,1 | 100 | 75,9 | 75,9 |
| $P_{1,4}Sb_{11,4}W_{4,8}Mo_{12}O_x$ | 48 | 1,57 | 377 | 1,1 | 100 | 76,1 | 76,1 |
| $P_{1,4}Sb_{11,4}W_{4,8}Mo_{12}O_x$ | 109 | 1,57 | 376 | 1,1 | 100 | 75,8 | 75,8 |

**Patentansprüche**

1. Verfahren zur Herstellung von Maleinsäureanhydrid durch Oxidation von $C_4$-Kohlenwasserstoffen, dadurch gekennzeichnet, dass man ein Gemisch aus ungesättigten n-$C_4$-Kohlenwasserstoffen und einem sauerstoffhaltigen Gas bei Temperaturen von 300 bis 450 °C und bei einer scheinbaren Kontaktzeit von 0,1 bis 30 Sekunden mit einem Katalysator in Berührung bringt, der die Formel

$W_aMo_bSb_cY_dX_eO_x$

hat, wobei Y Phosphor und/oder Silicium ist und X ein Element der Gruppe Vanadin, Wismuth, Tellur und/oder ein Alkalimetall sein kann und a und b Zahlen zwischen 1 und 12 sind, c eine Zahl zwischen 2 und 20, d eine Zahl zwischen 0,2 und 2, e eine Zahl zwischen 0 und 10 und x die Zahl der Sauerstoffatome ist, die für die Absättigung der restlichen Valenzen der übrigen vorhandenen Elemente erforderlich ist und den man durch Zusammengeben einer Verbindung des dreiwertigen Antimons sowie gegebenenfalls einer Vanadin-, Wismuth-, Tellur und/oder Alkaliverbindung mit einer Lösung der Heteropolysäuren des Molybdäns und Wolframs bei einer Temperatur von 0 bis 200 °C, anschliessende Trocknung und Temperung bei einer Temperatur von 300 bis 500 °C erhält.

2. Verfahren nach Anspruch 1, dadurch gekennzeichnet, dass Heteropolysäuren mit Phosphor und/oder Silicium als Heteroatome eingesetzt werden.

3. Verfahren nach Ansprüchen 1 und 2, dadurch gekennzeichnet, dass der Katalysator die Zusammensetzung
$W_{4,8}Mo_{12}P_{1,4}Sb_{11,4}O_x$ hat.

4. Verfahren nach Ansprüchen 1 bis 3, dadurch gekennzeichnet, dass der Katalysator die Zusammensetzung
$W_{4,8}Mo_{12}P_{1,4}Sb_{11,4}V_{1,1}Cs_{0,6}O_x$ hat.

5. Verfahren gemäss Ansprüchen 1 bis 4, dadurch gekennzeichnet, dass der Katalysator als Vollkontakt eingesetzt wird.

6. Verfahren gemäss Ansprüchen 1 bis 5, dadurch gekennzeichnet, dass die scheinbare Kontaktzeit 0,2 bis 10 Sekunden beträgt.

**Revendications**

1. Procédé pour la préparation d'anhydride maléique par oxydation d'hydrocarbures en $C_4$, caractérisé par le fait qu'on met en présence un mélange d'hydrocarbures n–$C_4$ non saturés et un gaz contenant de l'oxygène à des températures de 300 à 450 °C, pendant une durée de contact apparente de 0,1 à 30 secondes avec un catalyseur ayant pour formule

$W_aMo_bSb_cY_dX_eO_x$

Y étant du phosphore et/ou du silicium,
X un élément du groupe vanadine, bismuth, tellure et/ou un métal alcalin,
a et b des chiffres compris entre 1 et 12,
c un nombre compris entre 2 et 20,
d un nombre compris entre 0,2 et 2,
e un nombre compris entre 0 et 10 et
x le nombre d'atomes d'oxygène qui est nécessaire pour saturer les valences restantes des autres éléments présents et qu'on obtient par la mise en présence d'une combinaison de l'antimoine trivalent ainsi que le cas échéant d'une combinaison de vanadine, de bismuth, de tellure et/ou d'alcalin avec une solution des hétéropolyacides du molybdène et du tungstène à une température comprise entre 0 et 200 °C, séchage subséquent et recuit à une température de 300 à 500 °C.

2. Procédé selon la revendication 1, caractérisé par le fait que les hétéropolyacides sont mis en œuvre avec le phosphore et/ou le silicium comme hétéroatomes.

3. Procédé selon les revendications 1 et 2, caractérisé par le fait que le catalyseur a la composition

$W_{4,8}Mo_{12}P_{1,4}Sb_{11,4}O_x$

4. Procédé selon les revendications 1 à 3, caractérisé par le fait que le catalyseur a la composition

$W_{4,8}Mo_{12}P_{1,4}Sb_{11,4}V_{1,1}Cs_{0,6}O_x$

5. Procédé selon les revendications 1 à 4 caractérisé par le fait que le catalyseur est mis en œuvre en contact entier.

6. Procédé selon les revendications 1 à 5 caractérisé par le fait que la durée de contact apparents est comprise entre 0,2 et 10 secondes.

**Claims**

1. A process for the manufacture of maleic anhydride by oxidation of $C_4$-hydrocarbons, characterized in that a mixture of unsaturated n-$C_4$-hydrocarbons and an oxygen-containing gas are brought into ontact with a catalyst at a temperature of 300–450 °C for an apparent contact period of 0.1–30 seconds, the formula of the catalyst being

$W_aMo_bSb_cY_dX_eO_x$,

where Y=phosphorus and/or silicium and X=an element of the vanadium, bismuth, tellurium group, and/or an alkali metal; and a and b=numerical suffixes between 1 and 12; c=a numerical suffix between 2 and 20; d=a numerical suffix between 0.2 and 2; e=a numerical suffix between 0 and 10; and x=the number of oxygen atoms required to saturate the remaining valencies of other available elements, the catalyst being obtained by mixing a compound of the trivalent antimony, and if necessary, a vanadium, bismuth, tellurium and/or alkali compound with a solution of the heteropoly acids of molybdenum and tungsten at a temperature of 0–200 °C and subsequent drying and heat-treatment at a temperature of 300–500 °C.

2. A process of Claim 1, wherein heteropoly acids containing phosphorus and/or silicium are used as hetero atoms.

3. A process of Claim 1 and 2, wherein the catalyst has the following composition:
$W_{4.8}Mo_{12}P_{1.4}Sb_{11.4}O_x$.

4. A process of Claim 1–3, wherein the catalyst has the following composition:
$W_{4.8}Mo_{12}P_{1.4}Sb_{11.4}V_{1.1}Cs_{0.6}O_x$.

5. A process of Claim 1–4, wherein the catalyst consists entirely of active material.

6. A process of Claim 1–5, wherein the apparent contact period is 0.2–10 seconds.